# EUROPÄISCHE PATENTSCHRIFT

(11) **EP 3 682 715 B1**
(45) Veröffentlichungstag und Bekanntmachung des Hinweises auf die Patenterteilung: **09.03.2022**
(21) Anmeldenummer: 18772720.1
(22) Anmeldetag: 03.09.2018
(51) Int. Cl.: H05H 1/24, A46B 15/00

(54) **PLASMA-BEHANDLUNGSGERÄT UND PLASMABEHANDLUNGSSET MIT DIESEM**
PLASMA TREATMENT DEVICE AND PLASMA TREATMENT SET WITH THE SAME
APPAREIL DE TRAITEMENT AU PLASMA ET ENSEMBLE DE TRAITEMENT PAR PLASMA LE COMPRENANT

(30) Priorität: 11.09.2017 DE 102017120902
(43) Veröffentlichungstag der Anmeldung: 22.07.2020
(73) Patentinhaber: Cinogy GmbH, 37115 Duderstadt (DE)
(72) Erfinder: WANDKE, Dirk, 37308 Heilbad Heiligenstadt (DE); HAHNL, Mirko, 37339 Berlingerode (DE); STORCK, Karl-Otto, 37115 Duderstadt (DE); TRUTWIG, Leonhard, 37115 Duderstadt (DE); RICKE, Melanie, 37191 Katlenburg-Lindau (DE)
(74) Vertreter: Gramm, Lins & Partner Patent- und Rechtsanwälte PartGmbB
(86) Internationale Anmeldenummer: PCT/DE2018/100748
(87) Internationale Veröffentlichungsnummer: WO 2019/048001

(56) Entgegenhaltungen:
- WO-A1-2015/083155
- JP-A- 2015 080 489
- KR-A- 20170 063 261
- US-A1- 2016 193 475
- US-A1- 2016 338 814
- SUN SY ET AL: "Disinfecting gingival crevices by Non-thermal Atmospheric Pressure Plasma", MEDICAL DEVICES AND DIAGNOSTIC ENGINEERING, Bd. 1, Nr. 1, 5. September 2016 (2016-09-05), XP055524768, DOI: 10.15761/MDDE.1000101

## Beschreibung

Die Erfindung betrifft ein Plasma-Behandlungsgerät gemäß Anspruch 1 sowie ein Plasma-Behandlungsset gemäß Anspruch 10.

Bevorzugte Ausführungsformen der Erfindung sind in den abhängigen Ansprüchen 2-9 definiert.

Die Behandlung von Hautoberflächen, einschließlich Wundflächen, mit einem dielektrisch behinderten Plasma ist bekannt und erfährt eine zunehmende Aufmerksamkeit. Durch die Plasmabehandlung soll einerseits eine Keimreduzierung an der Haut- oder Wundoberfläche erfolgen und andererseits die Mikrozirkulation des Gewebes angeregt werden. Hierdurch wird beispielsweise eine Wundheilung begünstigt. Eine gesunde Haut kann durch die Behandlung kosmetisch verbessert werden. Darüber hinaus ist es möglich, die Plasmabehandlung mit der Zugabe von heilenden oder pflegenden Substanzen zu kombinieren, da durch die Plasmabehandlung die Aufnahme dieser Substanzen durch die Haut begünstigt wird.

Bei der Ausbildung eines dielektrisch behinderten Plasmas wird ein direkter Stromfluss durch eine dielektrische Sperrschicht unterbunden, sodass ein die Haut oder das Gewebe verbrennender Lichtbogen nicht entstehen kann.

Durch DE 103 24 926 B3 ist es grundsätzlich bekannt, die Plasmabehandlung mit einem dielektrisch behinderten Plasma an lebenden Zellen auszuführen. Ein Beispiel ist auch eine Zahnbehandlung. Hierfür wird eine zylindrische, stabförmige Elektrode verwendet, die von einem Dielektrikum umgeben ist und an ihrer Spitze ein Plasmafeld generiert, in dem der Elektrode Hochspannungsimpulse zugeführt werden und der Körper mit den zu behandelnden Zellen als Gegenelektrode fungiert. Dabei wird darauf geachtet, dass ein kleines Plasmafeld an der Spitze der Elektrode entsteht. Für die Behandlung einer größeren Oberfläche kann eine stabförmige Elektrode verwendet werden, die eine durch das Dielektrikum gebildete kreiszylindrische Mantelfläche aufweist. Dabei entsteht ein angenähert linienförmiges Plasmafeld. Um eine Fläche zu behandeln, wird die Elektrode über die zu behandelnde Fläche gerollt. Der Gedanke, eine größere Fläche durch Überrollen mit einer kugelförmigen Elektrode oder einer walzenförmigen Elektrode zu überstreichen, ist ferner Gegenstand der DE 10 2013 019 058 A1 und der EP 2 946 641 B1.

Durch WO 2011/076193 A1 ist es ferner bekannt, eine flexible Elektrode für eine flächige Behandlung einer Hautoberfläche auszubilden, mit der gleichmäßig eine größere Fläche nur das Auflegen der Elektrodenanordnung erfolgt. Dass die spannungsführende Elektrode umhüllende Dielektrikum liegt dabei auf einer Behandlungsseite unmittelbar an der Hautoberfläche an, ist aber auf dieser Seite so strukturiert, dass zwischen Anlagepunkten oder -linien Lufträume bestehen, in denen sich das Plasma ausbilden kann.

Alle bekannten Plasma-Behandlungsgeräte sind somit für die punktuelle, linienförmige oder flächige Oberflächenbehandlung ausgelegt. Dies gilt auch, soweit gemäß DE 103 24 926 B3 die Behandlung von Zähnen in der Mundhöhle in Erwägung gezogen worden ist. Hierfür ist ein Plasmafeld mit einer sehr geringen Ausdehnung vorgesehen, mit dem die Oberfläche eines Zahns überstrichen werden kann, um beispielsweise einen Kariesbefall zu behandeln.

Die US 2016/193475 A1 offenbart eine zur Behandlung von biologischem Gewebe mit einem Niederdruckplasma vorgesehene Vorrichtung offenbart. Die Sonde 2 bildet eine Metallelektrode 14 und ein Dielektrikum 15, um einen dielektrischen Barriereentladung erzeugen zu können. Eine flexible Anpassung der Elektrode an verschiedene komplizierte Kopfformen ist hiermit jedoch nicht möglich.

Die KR 10-2017/0063261 A offenbart einen Plasmajet, wobei ein Ventilator für eine Luft- oder Gasströmung im Griffteil angeordnet ist, wobei die das Plasma generierenden Elektroden jedoch im Kopfteil eingeordnet sind.

Der vorliegenden Erfindung liegt die Aufgabe zugrunde, ein Plasma-Behandlungsgerät, das für die Behandlung innerhalb der Mundhöhle eines Lebewesens vorgesehen ist, so auszubilden, dass eine vielfältige und verbesserte Behandlung möglich ist.

Zur Lösung dieser Aufgabe ist ein Plasma-Behandlungsgerät der eingangs erwähnten Art, wobei das Gehäuse ein Griffteil aufweist, an dem ein Kopfteil mittels einer mechanischen Verbindungsanordnung lösbar und auswechselbar befestigt ist, wobei das Griffteil alle zur Generierung der Hochspannungssignale erforderlichen Stufen der Hochspannungsstufe und eine Kontaktanordnung enthält, an der die Hochspannungssignale anliegen, dass das Kopfteil in einer für eine spezielle Behandlung speziellen Form ausgebildet ist und eine entsprechend geformte Elektrodenanordnung aufweist, die vollständig von dem Dielektrikum umgeben ist und einen Anschluss aufweist, der die Kontaktanordnung des Griffteils bei der mittels der mechanischen Verbindungsanordnung erfolgenden Befestigung des Kopfteils an dem Griffteil kontaktiert, erfindungsgemäß dadurch gekennzeichnet, dass die Elektrodenanordnung einstückig aus einem gießfähigen Kunststoff gebildet ist, dem elektrisch leitende Zusätze zugesetzt sind.

Das erfindungsgemäße Plasma-Behandlungsgerät weist die Besonderheit auf, dass die Elektrodenanordnung speziell für eine Behandlung innerhalb der Mundhöhle geformt ist. Die Plasmabehandlung von Zähnen, Zahnfleisch und Zunge war bisher nur punktuell möglich. Durch die vorliegende Erfindung wird das Kopfteil mit der Elektrodenanordnung so geformt, dass spezielle, bisher nicht mögliche Behandlungen überhaupt durchgeführt oder verbessert werden können. Darüber hinaus ist es durch die speziell geformten Kopfteile erreichbar, dass beispielsweise alle Zähne des Oberkiefers oder des Unterkiefers gemeinsam während desselben Behandlungsschritts behandelt werden können. Die erfindungsgemäße Auswechselbarkeit des Kopfteils aufgrund der lösbaren Verbindung zwischen Kopfteil und Griffteil erlaubt es ferner, eine umfassende Behandlung innerhalb der Mundhöhle mit demselben Griffteil und lediglich ausgewechselten Kopfteil nacheinander durchzuführen. Beispielsweise ist es möglich, zunächst die Zähne des Oberkiefers und/oder des Unterkiefers und anschließend mit einem anderen Kopfteil die Zahnzwischenräume zu behandeln. In einem anderen Ausführungsbeispiel können die Zahnoberflächen mit einem Kopfteil behandelt werden, während eine aufgrund eines Kariesbefalls durchgeführte Bohrung mit einem nadelförmig ausgebildeten Kopfteil behandelbar ist, das in die Bohrung eingeführt werden kann.

Gegenstand der Erfindung ist daher ferner ein Plasma-Behandlungsset, das aus einem Griffteil und wenigstens zwei verschieden geformten Kopfteilen besteht, die jeweils mit dem Griffteil lösbar verbindbar sind.

In einer Ausführungsform der Erfindung ist das Kopfteil nach Art einer Beißschiene mit einer Bodenwandung und zwei Seitenwandungen geformt, mit der die Zähne einer Kauleiste des Lebewesens umgreifbar sind, wobei die geformte Elektrode flächig in der Bodenwandung angeordnet ist. In dieser Ausführungsform sind die Zähne einer Kauleiste (Oberkiefer oder Unterkiefer) gemeinsam im selben Behandlungsschritt behandelbar. Durch die Ausformung des Kopfteils nach Art einer Beißschiene ist eine korrekte Positionierung der Elektrodenanordnung sichergestellt.

In einer anderen modifizierten Ausführungsform ist das Kopfteil mit einer ebenen Fläche in einer U-Form ausgebildet, die der Form einer Kauleiste entspricht. Mit diesem Kopfteil lassen sich insbesondere die Kauflächen der Zähne der Kauleiste behandeln.

In einer anderen Ausführungsform ist das Kopfteil als dünnes flaches Teil zum Einschieben in einen Zahnzwischenraum ausgebildet. Hierdurch werden insbesondere die zueinander zeigenden Zahnkanten benachbarter Zähne behandelt, was insbesondere beim Kariesbefall in dem Zahnzwischenraum angezeigt ist. Es ist ferner möglich, das Kopfteil in Form einer Zahnbürste auszubilden, die ggf. elektrisch bewegbar ist. Dadurch kann die Plasmabehandlung mit einer mechanischen Reinigung der Zähne kombiniert werden.

In einer anderen Ausführungsform weist das Kopfteil eine Rahmenform mit einer mittigen Öffnung auf, in die eine Zunge des Lebewesens hineinragen kann, wobei die Elektrodenanordnung sich über die gesamte Rahmenform erstreckt, also ebenfalls rahmenförmig ausgebildet ist. Dieses Kopfteil ist insbesondere dazu geeignet, einen bakteriellen Zungenbelag zu entfernen, wobei das rahmenförmige Kopfteil langsam über die Länge der Zunge mit einem proximalen Quersteg gezogen wird.

In einer weiteren Ausführungsform ist das Kopfteil in dünner Stabform in einer Größe ausgebildet, um in eine in einen Zahn vorgenommene Bohrung einführbar zu sein. Da sie die Elektrodenanordnung ebenfalls in dieser dünnen Stabform erstreckt, lässt sich eine in einem Zahn vorgenommene Bohrung mit einem derartigen Kopfteil mit einem dielektrisch behinderten Plasma behandeln.

In einer weiteren Ausführungsform der Erfindung ist das Kopfteil mit einem flächigen Bodenabschnitt mit einer Breite und einer Länge ausgebildet, aus dem sich über die Länge mehrere sich über die Breite erstreckende Stege erheben, die zum Einführen in Zahnzwischenräume ausgebildet sind. Das Kopfteil hat eine Ähnlichkeit mit der Borstenanordnung einer Zahnbürste, wobei die Stege vorzugsweise aus hochflexiblem dielektrischem Kunststoff bestehen. Hierfür eignet sich insbesondere Silikon. Die Stege können dann in Zahnzwischenräume zumindest teilweise eingedrückt werden, auch wenn der Abstand der Stege nicht vollständig mit dem Mittenabstand der Zahnzwischenräume übereinstimmt. Die Elektrodenanordnung ist dabei vorzugsweise für das Hineinragen in die Stege des Dielektrikums einstückig ausgebildet. Hierbei können neben den Stegen auch Borsten positioniert sein, mit denen die Plasmabehandlung mit einer mechanischen Reinigung kombiniert werden kann.

In allen Ausführungsformen kann die Elektrodenanordnung als eine Elektrode ausgebildet sein, die mit den Hochspannungsimpulsen beaufschlagt wird, wobei die zu behandelnden Teile der Mundhöhle als Gegenelektrode wirken. Die Elektrodenanordnung kann aber auch aus zwei voneinander isolierten Elektroden bestehen. In dieser Ausführungsform können die beiden Elektroden beide mit Hochspannungsimpulsen versorgt werden, die identisch, aber gegenphasig sind. Somit resultiert in einigem Abstand ein Nullfeld. Der zu behandelnde Teil der Mundhöhle dient auch hier als Gegenelektrode.

Bei der Ausbildung der Elektrodenanordnung mit zwei Elektroden ist es ferner möglich, eine der Elektroden mit den Hochspannungssignalen und die andere mit einem Bezugspotential zu verbinden. In diesem Fall bildet sich das Plasmafeld nicht zwischen der Elektrodenanordnung und dem zu behandelnden Teil der Mundhöhle als Gegenelektrode aus, sondern zwischen den beiden Elektroden. Da sich die Feldlinien bekanntlich am Rand der Elektroden bogenförmig erstrecken, kann in dieser Anordnung ein Oberflächenplasma erzeugt werden, das allerdings weniger effizient gebildet wird.

Die Erfindung soll im Folgenden anhand von in der Zeichnung dargestellten Ausführungsbeispielen näher erläutert werden. Es zeigen:
- Figur 1: Darstellungen eines Ausführungsbeispiels mit einer ersten Ausführungsform eines Kopfteils, das lösbar am Griffteil des Plasma-Be-handlungsgeräts befestigbar ist;
- Figur 2: Schnittdarstellungen analog der Schnittlinie A-A in Figur 1b) für sechs weitere Ausführungsbeispiele von Kopfteilen, die mit dem in Figur 1 dargestellten Griffteil verbindbar sind;
- Figur 3: Draufsichten auf die Oberseiten der sieben in Figur 1 und 2 dargestellten Kopfteile;
- Figur 4: rückwärtige, vom Griffteil aus gesehene Ansichten der sieben Kopfteile gemäß Figur 3;
- Figur 5: Ansichten der Unterseiten der Kopfteile gemäß Figur 3;
- Figur 6: rückwärtige Ansichten gemäß Figur 4 mit Darstellung einer Schnittlinie B-B;
- Figur 7: Horizontalschnitte durch die sieben Ausführungsformen von Kopfteilen entlang der Schnittlinie B-B aus Figur 6;
- Figur 8: eine Schnittdarstellung gemäß Figur 7, eine Seitenansicht gemäß Figur 1 und eine Draufsicht gemäß Figur 3 eines modifizierten Kopfteils;
- Figur 9: Darstellungen gemäß Figur 8 für ein weiter modifiziertes Kopfteil.

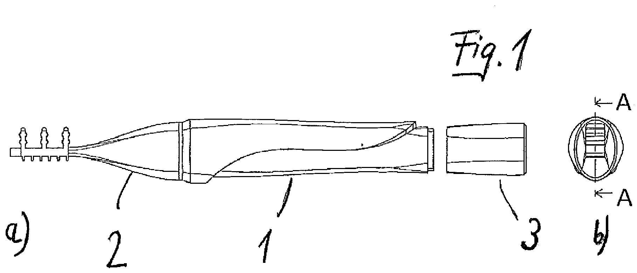

Figur 1 zeigt ein komplettes Plasma-Behandlungsgerät in einer Seitenansicht (Figur 1a)), in einer stirnseitigen Ansicht auf das Kopfteil gesehen (Figur 1b)) sowie in einem Hochschnitt entlang der Schnittlinie A-A aus Figur 1b) (Figur 1c)). Das dargestellte Ausführungsbeispiel eines Plasma-Behandlungsgeräts weist ein Gehäuse auf, das aus einem Griffteil 1, einem Kopfteil 2 und einem rückwärtigen Abschlussteil 3 zusammengesetzt ist. Die Verbindungen zwischen Griffteil 1 und Kopfteil 2 sowie zwischen Griffteil 1 und rückwärtigem Abschlussteil 3 sind vorzugsweise Schnappverbindungen, können aber auch als Schraubverbindungen, Bajonettverbindungen o. ä. ausgebildet sein. Das Griffteil 1 weist eine leicht ergonomische geformte Außenkontur auf, die das sichere Greifen des Plasma-Behandlungsgeräts ermöglichen soll. Das hohle Griffteil 1 weist einen Innenraum 4 auf, in dem sich eine elektronische Steuerung 5 befindet, die aus einer Gleichspannung Wechselspannungsimpulse generiert, die mit einer Spulenanordnung 6 in Hochspannungsimpulse übersetzt werden. Steuerung 5 und Spulenanordnung 6 bilden somit eine Hochspannungsstufe 5, 6. Die Hochspannungsimpulse gelangen auf eine in ein stirnseitiges isolierendes Abschlussstück 7 eingesetzte, nicht näher dargestellte Kontaktanordnung in Form einer Buchse, über die die generierten Hochspannungsimpulse auf das Kopfteil 2 übertragen werden können.

Das rückwärtige Abschlussteil 3 umschließt drei Batterien 8, die durch Abnahme des rückwärtigen Abschlussteils 3 leicht zugänglich und auswechselbar sind. Das in Figur 1 dargestellte Ausführungsbeispiel stellt somit ein Handgerät dar, das ohne Zuleitungskabel auskommt und daher bequem handhabbar ist. Im Rahmen der Erfindung ist es allerdings auch denkbar, das Gerät mit einer Stromzuführung über ein Kabel zu versorgen, wobei die Stromversorgung eine Gleichspannungsversorgung oder eine Wechselspannungsversorgung, beispielsweise aus dem öffentlichen Stromnetz, sein kann. Im letztgenannten Fall enthält die Steuerung 5 zweckmäßigerweise eine Gleichrichter- und Zerhackerstufe.

In dem in Figur 1 dargestellten Ausführungsbeispiel ist das Kopfteil 2 als speziell geformtes Kopfteil 2a mit einem einstückigen Dielektrikum 9 ausgebildet, das eine Elektrodenanordnung 10 vor einer Berührung abschirmt, wenn das Kopfteil 2a mit dem Griffteil 1 ordnungsgemäß verbunden ist. Das Dielektrikum 9 ist für das Kopfteil 2a so geformt, dass es von einer etwa zylindrischen Form des Griffteils 1 ausgehend sich verjüngt und in einen flächigen Bodenabschnitt 11 übergeht, von dem aus sich drei Stege 12 nach oben erstrecken und zwischen sich Zwischenräume 13 ausbilden. Die Elektrodenanordnung bildet am zum Griffteil 1 zeigenden Ende einen leitenden Anschluss 14 in Form eines aus dem Dielektrikum 9 herausstehenden Anschlussstifts aus, der in die erwähnte Buchse im Abschlussstück 7 des Griffteils 1 einsteckbar ist und so den Kontakt zur Spulenanordnung 6 herstellt. Die Elektrodenanordnung 10 erstreckt sich ausgehend von dem Anschluss 14 in den flächigen Bodenabschnitt 11 hinein und bildet dort einen flächigen Elektrodenabschnitt 15. Mit diesem flächigen Elektrodenabschnitt 15 sind Stegansätze 16 einstückig verbunden, die sich zentral in die Stege 12 erstrecken und allseits von dem Dielektrikum der Stege 12 umschlossen sind. Die komplizierte Form der Elektrodenanordnung 10 lässt sich erfindungsgemäß einstückig ausbilden, wenn die Elektrodenanordnung aus einem gießfähigen Kunststoff gebildet wird, dem elektrisch leitende Zusätze, vorzugsweise in Partikelform, zugesetzt sind. Ein geeignetes gießfähiges Material ist ein Silikon, das sich mit einem als Dielektrikum verwendeten elektrisch isolierenden Silikon materialschlüssig verbindet.

Die Stege 12 dienen zur Positionierung des Kopfteils 2a in Zahnzwischenräumen, in die sie - zumindest teilweise - eindrückbar sind. Die Stege 12 bestehen aus einem weichflexiblen Material und können daher in Zahnzwischenräume auch eindringen, wenn die Breite des Zahns nicht vollständig mit der Breite des Zwischenraums 13 zwischen den Stegen 12 übereinstimmt. Da die Elektrodenanordnung 10 mit dem Elektrodenabschnitt 15 und den Stegansätzen 16 den Zahn von drei Seiten umgeben kann, wird eine bestmöglich vollständige Plasmabehandlung der zwischen den Stegen 12 befindlichen Zähne ermöglicht.

Wie in Figur 1 dargestellt ist, kann das Dielektrikum im Bereich der Stege 12 beispielsweise mit einem sägezahnförmigen Profil ausgebildet sein, um das Eindringen in Zahnzwischenräume zu erleichtern.

Auf der von den Stegen 12 abgewandten Rückseite des Bodenabschnitts 11 können quer zur Längsrichtung des Kopfteils 2a Verstärkungsstege 17 beziehungsweise Schwächungsnuten vorgesehen sein, die die Elastizität des Dielektrikums 9 im Bereich des Bodenabschnitts 11 erhöhen.

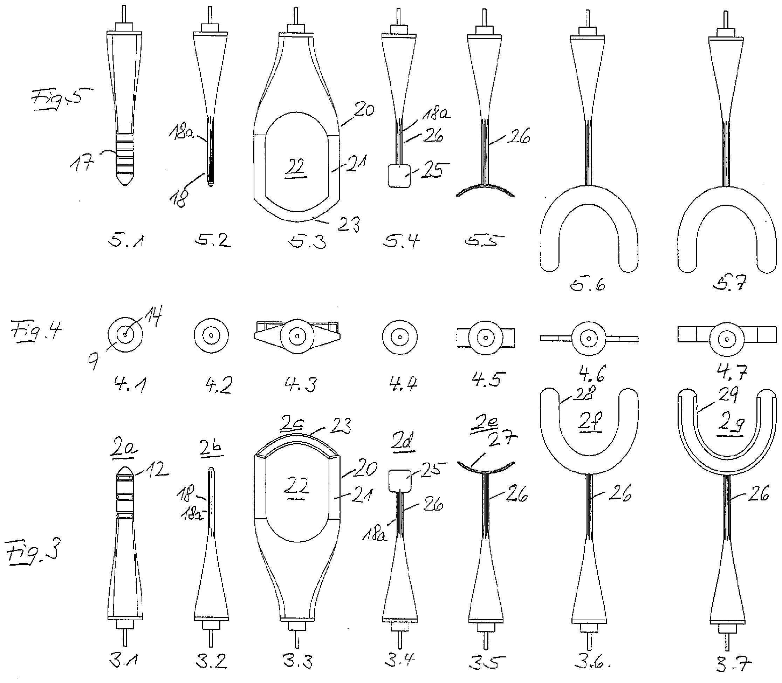

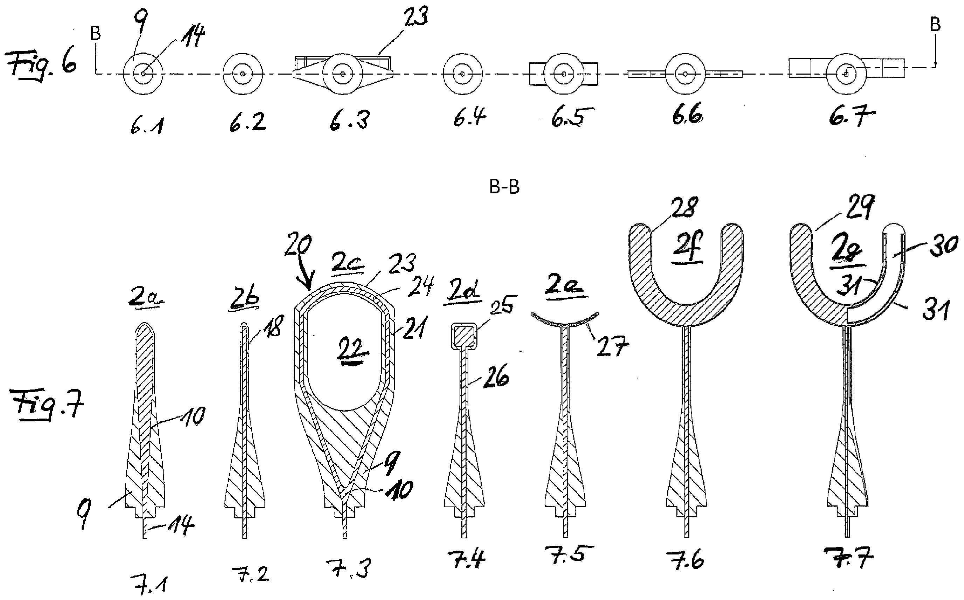

Das erste Ausführungsbeispiel eines Kopfteils 2a ist in den Figuren 3 bis 7 in verschiedenen Ansichten und einer weiteren Schnittdarstellung in den Abbildungen 3.1 bis 7.1 dargestellt.

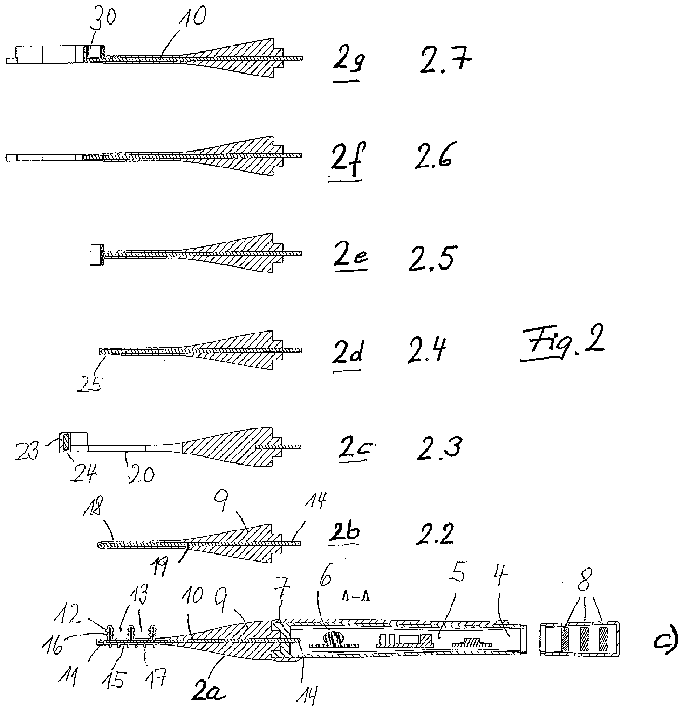

Ein Kopfteil 2b in einer zweiten Ausführungsform ist in den Figuren 2 bis 7 jeweils in den Abbildungen 2.2 bis 7.2 dargestellt. Das Dielektrikum 9 erstreckt sich von dem Anschlussstück zum Griffteil 1 ausgehend über eine Verjüngung in ein dünnes stabförmiges Ende 18, dessen Durchmesser unter 1 mm liegt und das mit Längsrillen 18a versehen ist, die einerseits eine mechanische Verstärkung bewirken und andererseits beim Anliegen, beispielsweise an einem Zahn, Lufträume begrenzen, in denen das Plasma entstehen kann. Die Elektrodenanordnung 10 erstreckt sich von dem Anschluss 14 stiftförmig in das stabförmige Ende 18 hinein und bildet eine drahtähnliche Elektrodenseele 19 für das stabförmige Ende 18. Am stirnseitigen Ende ist die Elektrodenseele 19 selbstverständlich durch das Dielektrikum abgedeckt, hier mit einem abgerundeten Ende.

Eine dritte Ausführungsform eines Kopfteils 2c ist in den Figuren 2 bis 7 mit den Abbildungen 2.3 bis 7.3 dargestellt. In diesem Fall verjüngt sich das Dielektrikum 9 zu einem Flachstück 20, das einen Rahmen 21 mit einer zentralen Öffnung 22 ausbildet. Die Öffnung wird am bezüglich des Griffteils 1 distalen Ende durch einen erhöhten Abschlusssteg 23 geschlossen. Die Öffnung 22 ist so dimensioniert, dass sie zumindest einen Teil der Zunge aufnehmen kann. Der Abschlusssteg 23 bildet eine Schabekante, mit der das Kopfteil 2c über die Zunge gezogen werden kann, um beispielsweise einen bakteriellen Belag der Zunge zu entfernen. Die Elektrodenanordnung 10 bildet auch hier den Anschluss 14 aus und gabelt sich innerhalb des Dielektrikums 9, um in der Ebene des Flachstücks 20 mittig in der Höhe des Dielektrikums 9 die Öffnung 22 zu umschließen, wobei im Bereich des Abschlussstegs 23 auch die Elektrodenanordnung 10 zu einem erhöhtem Steg 24 geformt ist. Das Kopfteil 2 ist somit speziell für die Behandlung der Zunge geformt.

In einer vierten Ausführungsform ist ein Kopfteil 2d als flaches ebenes Endstück 25 ausgebildet, das sich an einem schmalen und daher hochelastischen stabförmigen Übergangsstück 26 ebenfalls mit Längsrillen 18a befindet. Dieses Kopfteil eignet sich speziell für die Behandlung von befallenen Zahnzwischenräumen, da die flächige Elektrode in dem Endstück 25 auf beiden Seiten des Endstücks ein konzentriertes Plasmafeld entstehen lässt, das auf die angrenzenden Zähne einwirkt, wenn das Kopfteil 2d in einen Zahnzwischenraum eingeführt ist.

Gemäß der in den Figuren 2 bis 7 in den Abbildungen 2.5 bis 7.5 dargestellten fünften Ausführungsform weist das Kopfteil 2e ebenfalls ein stabförmiges Übergangsstück 26 auf, an das sich ein bogenabschnittförmiges Endstück 27 anschließt. Das Endstück steht senkrecht zu der durch die Elektrodenanordnung 10 gebildeten Ebene und ist insbesondere geeignet, Außenbereiche des Kiefers, insbesondere am Übergang zwischen Zahnfleisch und Zahn bereichsweise zu behandeln.

In einer sechsten Ausführungsform, dargestellt in den Abbildungen 2.6 bis 7.6 der Figuren 2 bis 7, weist das im Übrigen in gleicher Weise wie beim vierten und fünften Ausführungsbeispiel ausgeführte Kopfteil 2f ein U-förmiges Endstück auf, das in der Ebene der Elektrodenanordnung 10 einen U-förmigen Streifen bildet. Das Kopfteil 2f eignet sich daher insbesondere zur Behandlung der Kauflächen und - Kanten von Oberkiefer und Unterkiefer, wenn die Zähne an den U-förmigen Endstück an Oberseite und Unterseite anliegen.

In einem siebten Ausführungsbeispiel, dargestellt in den Abbildungen 2.7 bis 7.7 der Figuren 2 bis 7, weist das Kopfteil 2g ein Endstück 29 nach Art einer Beißschiene auf, sodass das Dielektrikum 9 einen nach oben offenen Kanal mit einer Bodenwandung 30 und zwei Seitenwandungen 31 bildet. Die Elektrodenanordnung 10 erstreckt sich zumindest über die Bodenwandung, kann sich aber auch in die Seitenwandungen hinein erstrecken. Dieses Kopfteil 2g ist für die Behandlung der gesamten Zähne eines Oberkiefers oder eines Unterkiefers geeignet.

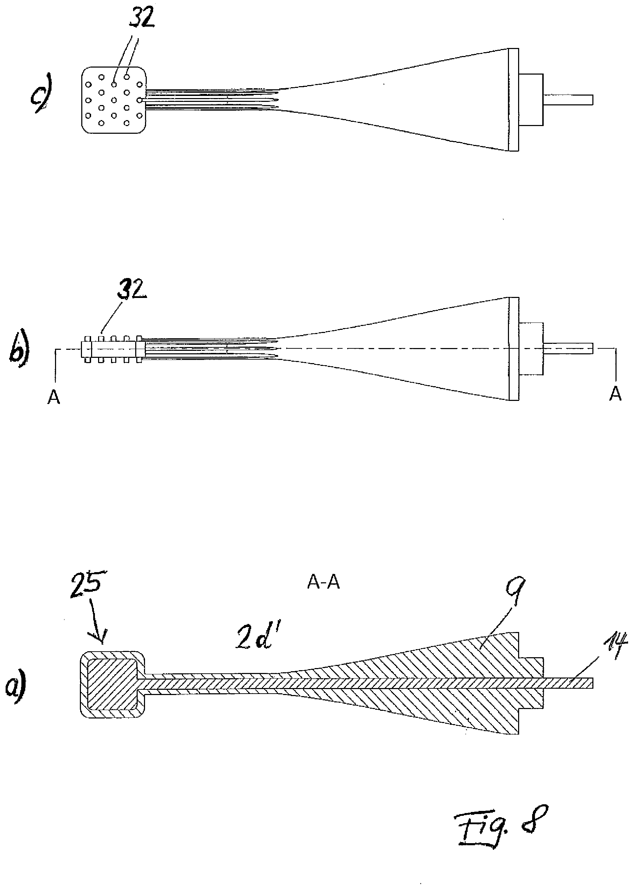

Figur 8 zeigt in vergrößerter Darstellung einen Horizontalschnitt (Figur 8a), eine Seitenansicht (Figur 8b) und eine Draufsicht (Figur 8c) eines modifizierten Kopfteils 2d'. Das modifizierte Kopfteil 2d' unterscheidet sich von dem Kopfteil 2d in den Figuren 2.4 bis 7.4 dadurch, dass das Dielektrikum 9 an der Oberfläche des flachen ebenen Endstücks 25 mit kleinen Noppen 32 ausgebildet ist, mit denen das flache ebene Endstück 25 an einem zu behandelnden Körperteil anliegen kann, wobei zwischen den Noppen 32 Lufträume bestehen, in denen sich das Plasma ausbilden kann. Die Noppen 32 bilden lediglich ein Ausführungsbeispiel für eine strukturierte Oberfläche, die auch an den anderen Kopfteilen 2 realisiert sein können, auch in Form einer gitterartigen Struktur, einer Porenstruktur usw. Eine gleiche Funktion erfüllen die Verstärkungsrippen 17 am Kopfteil 2a und die Längsrillen 18a an den Ausführungsformen der Kopfteile 2b, 2d, 2e, 2f und 2g. Es ist ersichtlich, dass die Noppen 32 nicht auf eine bestimmte Anordnung festgelegt sind, sodass die Darstellung in Figur 8c nur zur Veranschaulichung eines Beispiels dient.

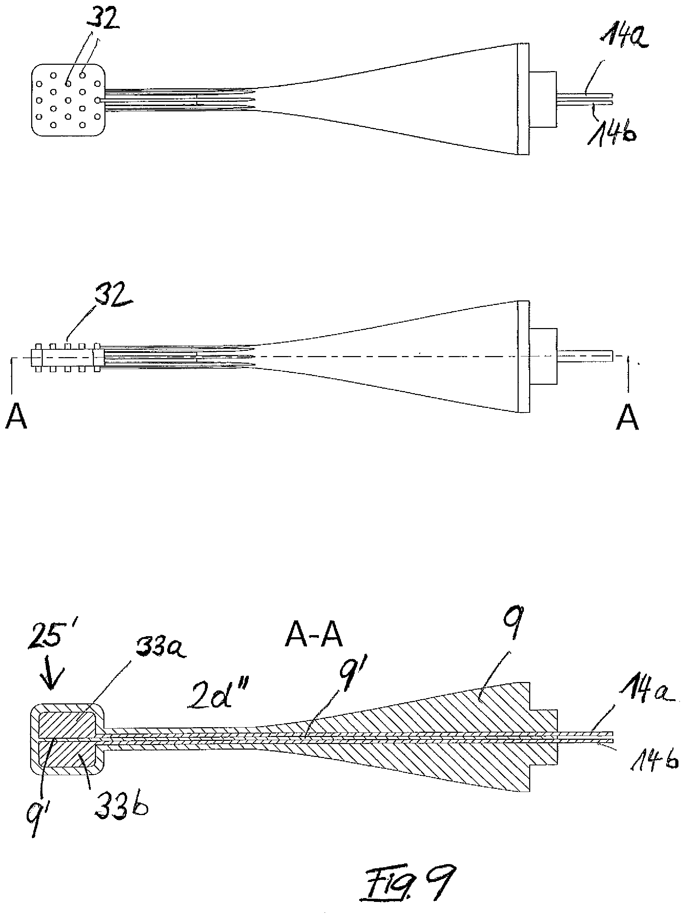

Figur 9 zeigt Ansichten wie in Figur 8 für ein weiter modifiziertes Kopfteil 2d". Während die Ausführungsform gemäß Figur 8 eine einzige Elektrode in dem Flachstück 25 aufweist, enthält das Kopfteil 2d" in dem flachen ebenen Endstück 25' zwei nebeneinander liegende Elektrodenflächen 33a, 33b, die über parallel verlaufende Anschlussleitungen 14a, 14b in dem Dielektrikum 9 an die benötigte Hochspannung anschließbar sind. Die beiden Anschlussleitungen 14a, 14b sind ebenso wie die Elektrodenflächen 33a, 33b durch einen mittigen Dielektrikumsstreifen 9' voneinander isoliert.

Bevorzugt werden die beiden Elektrodenflächen 33a und 33b mit identischen Hochspannungsimpulssignalen versorgt, die jeweils zueinander gegenpolig sind, sodass sich bereits in einem geringen Abstand die gebildeten Felder auslöschen und nur im Nahbereich das Plasma entsteht. Im Übrigen entspricht das Kopfteil 2d" äußerlich dem Kopfteil 2d' und ist bevorzugt ebenfalls mit Noppen 32 oder einer anderen Strukturierung des Dielektrikums 9 versehen.

Die dargestellten Ausführungsbeispiele verdeutlichen, dass mit einem Gerät durch den Austausch der Kopfteile 2 unterschiedliche Plasmabehandlungen in der Mundhöhle durchgeführt werden können, weil die Kopfteile 2 an die jeweilige Behandlungsmaßnahme angepasste Formen aufweisen.

## Patentansprüche

1. Plasma-Behandlungsgerät, ausgebildet für eine Behandlung innerhalb der Mundhöhle eines Lebewesens mit einem dielektrisch behinderten Plasma, mit einer in einem Gehäuse angeordneten Hochspannungsstufe (5, 6) zur Generierung von für die Erzeugung des Plasmas erforderlichen Hochspannungssignalen und einer an die Hochspannungsstufe angeschlossenen Elektrodenanordnung (10), die von einem Dielektrikum (9) zur Bildung eines dielektrisch behinderten Plasmas abgedeckt ist, wobei das Gehäuse ein Griffteil (1) aufweist, an dem ein Kopfteil (2) mittels einer mechanischen Verbindungsanordnung lösbar und auswechselbar befestigt ist, dass das Griffteil (1) alle zur Generierung der Hochspannungssignale erforderlichen Stufen der Hochspannungsstufe (5, 6) und eine Kontaktanordnung enthält, an der die Hochspannungssignale anliegen, wobei das Kopfteil (2) in einer für eine spezielle Behandlung speziellen Form ausgebildet ist und eine entsprechende geformte Elektrodenanordnung (10) aufweist, die vollständig von dem Dielektrikum (9) umgeben ist und einen Anschluss (14) aufweist, der die Kontaktanordnung des Griffteils (1) bei der mittels der mechanischen Verbindungsanordnung erfolgenden Befestigung des Kopfteils (2) an dem Griffteil (1) kontaktiert, **dadurch gekennzeichnet, dass** die Elektrodenanordnung (10) einstückig aus einem gießfähigen Kunststoff gebildet ist, dem elektrisch leitende Zusätze zugesetzt sind.

2. Plasma-Behandlungsgerät nach Anspruch 1, **dadurch gekennzeichnet, dass** das Kopfteil (2g) nach Art einer Beißschiene mit einer Bodenwandung (30) und zwei Seitenwandungen (31) geformt ist, mit der die Zähne einer Kauleiste des Lebewesens umgreifbar sind und dass die geformte Elektrodenanordnung (10) zumindest flächig in der Bodenwandung (30) angeordnet ist.

3. Plasma-Behandlungsgerät nach Anspruch 1, **dadurch gekennzeichnet, dass** das Kopfteil (2f) mit einer ebenen Fläche mit einem U-förmigen Endstück ausgebildet ist, das der Form einer Kauleiste entspricht.

4. Plasma-Behandlungsgerät nach Anspruch 1, **dadurch gekennzeichnet, dass** das Kopfteil (2d) als dünnes flaches Endstück (25) zum Einschieben in einen Zahnzwischenraum ausgebildet ist.

5. Plasma-Behandlungsgerät nach Anspruch 1, **dadurch gekennzeichnet, dass** das Kopfteil (2c) eine Rahmenform (21) mit einer mittigen Öffnung (22) aufweist, in die eine Zunge des Lebewesens zumindest teilweise hineinragen kann und dass die Elektrodenanordnung (10) eine entsprechende Rahmenform aufweist.

6. Plasma-Behandlungsgerät nach Anspruch 1, **dadurch gekennzeichnet, dass** das Kopfteil (2b) in dünner Stabform in einer Größe ausgebildet ist, um in eine in einen Zahn vorgenommene Bohrung einführbar zu sein.

7. Plasma-Behandlungsgerät nach Anspruch 1, **dadurch gekennzeichnet, dass** das Kopfteil (2a) mit einem flächigen Bodenabschnitt (11) mit einer Breite und einer Länge ausgebildet ist, aus dem sich über die Länge mehrere, sich über die Breite erstreckende Stege (12) erheben, die zum Einführen in Zahnzwischenräume ausgebildet sind.

8. Plasma-Behandlungsgerät nach Anspruch 7, **dadurch gekennzeichnet, dass** sich die Elektrodenanordnung (10) flächig in dem Bodenabschnitt (11) und einstückig in die Stege (12) hinein erstreckt.

9. Plasma-Behandlungsgerät nach Anspruch 7 oder 8, **dadurch gekennzeichnet, dass** das Kopfteil (2a) als Zahnbürste ausgebildet ist.

10. Plasma-Behandlungsset, gebildet mit einem Plasma-Behandlungsgerät nach Anspruch 1 und mit wenigstens zwei verschiedenen Kopfteilen (2).

## Claims

1. Plasma treatment device designed for a treatment within the oral cavity of a living being using a dielectric barrier plasma, comprising a high-voltage stage (5, 6), which is arranged in a housing and serves to generate high-voltage signals required for generating the plasma, and comprising an electrode arrangement (10), which is connected to the high-voltage stage and is covered by a dielectric (9) for forming a dielectric barrier plasma, wherein the housing has a handle part (1), to which a head part (2) is fastened in a releasable and exchangeable manner by means of a mechanical connection arrangement, the handle part (1) contains all the stages of the high-voltage stage (5, 6) that are required for generating the high-voltage signals and also contains a contact arrangement, at which the high-voltage signals are applied, wherein the head part (2) is designed in a shape specific to a specific treatment and has a corresponding shaped electrode arrangement (10), which is surrounded completely by the dielectric (9) and has a terminal (14) which makes contact with the contact arrangement of the handle part (1) when the head part (2) is fastened to the handle part (1) by means of the mechanical connection arrangement, **characterized in that** the electrode arrangement (10) is formed integrally from a moldable plastic, to which electrically conductive additives are added.

2. Plasma treatment device according to Claim 1, **characterized in that** the head part (2g) is shaped in the manner of a dental splint with a base wall (30) and two side walls (31), which makes it possible to engage around the teeth of a series of teeth of the living being, and **in that** the shaped electrode arrangement (10) is arranged at least areally in the bottom wall (30).

3. Plasma treatment device according to Claim 1, **characterized in that** the head part (2f) is designed with a planar area having a U-shaped end piece, which corresponds to the shape of a series of teeth.

4. Plasma treatment device according to Claim 1, **characterized in that** the head part (2d) is designed as a thin flat end piece (25) for insertion into an interdental space.

5. Plasma treatment device according to Claim 1, **characterized in that** the head part (2c) has a frame shape (21) with a central opening (22) into which a tongue of the living being can at least partially project, and **in that** the electrode arrangement (10) has a corresponding frame shape.

6. Plasma treatment device according to Claim 1, **characterized in that** the head part (2b) is designed in the shape of a thin rod, which is of a size allowing it to be introduced into a hole made in a tooth.

7. Plasma treatment device according to Claim 1, **characterized in that** the head part (2a) is designed with an areal base portion (11) having a width and a length, from which base portion (11) there rise, over the length thereof, a plurality of ridges (12) which extend over the width and are designed for insertion into interdental spaces.

8. Plasma treatment device according to Claim 7, **characterized in that** the electrode arrangement (10) extends areally in the base portion (11) and integrally into the ridges (12).

9. Plasma treatment device according to Claim 7 or 8, **characterized in that** the head part (2a) is designed as a toothbrush.

10. Plasma treatment set, formed with a plasma treatment device according to Claim 1 and with at least two different head parts (2).

## Revendications

1. Appareil de traitement au plasma, réalisé pour un traitement à l'intérieur de la cavité buccale d'un être vivant avec un plasma à barrière diélectrique, comportant un étage haute tension (5, 6) disposé dans un boîtier et destiné à générer des signaux haute tension nécessaires à la production du plasma, et un ensemble d'électrode (10) raccordé à l'étage haute tension et recouvert d'un diélectrique (9) pour former un plasma à barrière diélectrique,
dans lequel le boîtier comporte une partie formant poignée (1) à laquelle une partie de tête (2) est fixée de manière amovible et interchangeable au moyen d'un ensemble de liaison mécanique,
la partie formant poignée (1) comprend tous les étages de l'étage haute tension (5, 6) qui sont nécessaires pour générer les signaux haute tension, et un ensemble de contact auquel sont appliqués les signaux haute tension,
la partie de tête (2) est réalisée en une forme spéciale pour un traitement particulier et comprend un ensemble d'électrode (10) formé de manière correspondante, qui est entièrement entouré par le diélectrique (9) et qui comprend un raccord (14) qui entre en contact avec l'ensemble de contact de la partie formant poignée (1) lors de la fixation de la partie de tête (2) à la partie formant poignée (1) au moyen de l'ensemble de liaison mécanique,
**caractérisé en ce que**
l'ensemble d'électrode (10) est formé d'un seul tenant en une matière plastique capable de couler, à laquelle sont ajoutés des additifs électriquement conducteurs.

2. Appareil de traitement au plasma selon la revendication 1,
**caractérisé en ce que** la partie de tête (2g) est formée à la manière d'une gouttière de morsure comprenant une paroi de fond (30) et deux parois latérales (31) et permettant d'entourer les dents d'une barre de mastication de l'être vivant, et
**en ce que** l'ensemble d'électrode (10) formé est disposé au moins à plat dans la paroi de fond (30).

3. Appareil de traitement au plasma selon la revendication 1,
**caractérisé en ce que** la partie de tête (2f) est réalisée avec une surface plane ayant un embout d'extrémité en forme de U qui correspond à la forme d'une barre de mastication.

4. Appareil de traitement au plasma selon la revendication 1,
**caractérisé en ce que** la partie de tête (2d) est réalisée sous la forme d'un embout d'extrémité plat mince (25) destiné à être inséré dans un espace interdentaire.

5. Appareil de traitement au plasma selon la revendication 1,
**caractérisé en ce que** la partie de tête (2c) présente une forme de cadre (21) avec une ouverture centrale (22) dans laquelle une langue de l'être vivant peut pénétrer au moins partiellement, et
**en ce que** l'ensemble d'électrode (10) présente une forme de cadre correspondante.

6. Appareil de traitement au plasma selon la revendication 1,
**caractérisé en ce que** la partie de tête (2b) est réalisée sous la forme d'une tige mince d'une taille permettant de l'insérer dans un trou percé dans une dent.

7. Appareil de traitement au plasma selon la revendication 1,
**caractérisé en ce que** la partie de tête (2a) est réalisée avec une portion de fond plate (11) ayant une largeur et une longueur, à partir de laquelle s'élèvent sur la longueur plusieurs nervures (12) s'étendant sur la largeur, qui sont réalisées pour être introduites dans des espaces interdentaires.

8. Appareil de traitement au plasma selon la revendication 7,
**caractérisé en ce que** l'ensemble d'électrode (10) s'étend à plat jusque dans la portion de fond (11) et d'un seul tenant jusque dans les nervures (12).

9. Appareil de traitement au plasma selon la revendication 7 ou 8,
**caractérisé en ce que** la partie de tête (2a) est réalisée comme une brosse à dents.

10. Kit de traitement au plasma formé avec un appareil de traitement au plasma selon la revendication 1 et avec au moins deux parties de tête (2) différentes.
